# EUROPÄISCHE PATENTSCHRIFT

(11) **EP 0 402 669 B1**
(45) Veröffentlichungstag und Bekanntmachung des Hinweises auf die Patenterteilung: **12.01.1994**
(21) Anmeldenummer: 90109825.1
(22) Anmeldetag: 23.05.1990
(51) Int. Cl.: B65D 25/08

(54) **Behälter für durch Mischen von Komponenten herzustellende Substanzen**
Container for substances produced by mixing of components
Récipient pour substances préparées par mélange des composants

(30) Priorität: 15.06.1989 DE 8907336 U
(43) Veröffentlichungstag der Anmeldung: 19.12.1990
(73) Patentinhaber: THERA Patent GmbH & Co. KG Gesellschaft für industrielle Schutzrechte, D-82229 Seefeld (DE)
(72) Erfinder: Herold, Wolf Dietrich, Dr., D-8031 Seefeld 2 (DE); Koran, Peter, Dr., D-8120 Weilheim (DE); Pauser, Helmut, D-8918 Diessen (DE)
(74) Vertreter: Strehl Schübel-Hopf Groening & Partner

(56) Entgegenhaltungen:
- EP-A- 0 251 193
- DE-A- 3 208 472
- GB-A- 793 277
- US-A- 3 885 710

## Beschreibung

Zur Herstellung von Stoffgemischen aus zwei chemisch miteinander reagierenden Komponenten, insbesondere einer flüssigen und einer pulverförmigen Komponente, ist aus DE-A-2 009 403 ein Behälter gemäß dem Oberbegriff des Anspruchs 1 bekannt, der zwei im Lagerzustand gegeneinander verschlossene Kammern zur getrennten Aufnahme der beiden Komponenten aufweist. Zur Einleitung des Mischvorgangs wird durch Druck auf die Flüssigkeitskammer eine zwischen den beiden Kammern vorhandene Trennfolie durchbrochen und die Flüssigkeit durch fortgesetzte Druckausübung aus ihrer Kammer verdrängt und in die das Pulver enthaltende Kammer überführt, in der durch auf den Behälter einwirkende Schüttelbewegungen der eigentliche Mischvorgange erfolgt. Ähnliche Mischbehälter sind in DE-A-1 939 316, DE-B-1 287 251, DE-A-2 060 626 und DE-A-3 545 614 beschrieben.

In US-A-3 370 754 und DE-A-3 715 682 sind ferner Vorrichtungen zum Mischen und Ausbringen von Substanzen in Form von Zwei-Kammer-Mischbehältern offenbart, bei denen die Trennung zwischen den Kammern jeweils eine in der Wand der Mischkammer angeordnete Ventileinrichtung umfaßt. Jede dieser Druckschriften zeigt die im ersten Teil des Anspruchs 1 angegebenen Merkmale.

Weitere Misch- und Ausbringgeräte für Zwei-Komponenten-Substanzen, die jeweils ein ventilartiges Element zur Trennung der beiden Komponenten im Lagerzustand aufweisen, sind aus US-A-3 885 710, US-A-3 699 961 und US-A-3 477 431 bekannt.

Vom Hersteller werden die Komponenten in solchen Mengen vorgegeben, daß die fertig gemischte Substanz optimale Eigenschaften aufweist. Ändert sich das Mischungsverhältnis, so verschlechtern sich die Eigenschaften. Diese Möglichkeit ist bei den bekannten Behältern nicht ausgeschlossen, da die Gefahr besteht, daß infolge unvollständiger Volumenverkleinerung der Flüssigkeitskammer die Flüssigkeit nicht vollständig in die Mischkammer hinein entleert wird oder ein Teil der Flüssigkeit beim Mischvorgang in das gegebenenfalls noch vorhandene Volumen der Flüssigkeitskammer zurückfließt. Eine weitere Schwierigkeit besteht bei den bekannten Mischbehältern darin, daß Teile der Folie in das Substanzgemisch gelangen können.

Der Erfindung liegt die Aufgabe zugrunde, einen Behälter für durch Mischen von Komponenten herzustellende Substanzen anzugeben, der die Einhaltung des jeweils vorgegebenen Mischungsverhältnisses innerhalb der fertigen Substanz in hohem Maße gewährleistet.

Die erfindungsgemäße Lösung dieser Aufgabe ist im Anspruch 1 gekennzeichnet. Der danach ausgebildete Behälter eignet sich insbesondere zum Einsatz in ein kombiniertes Zentrifugen- und Mischgerät, bei dem die Überführung der ersten Komponente in die Mischkammer durch eine auf die erste Komponente einwirkende, zur Mischkammer gerichtete Zentrifugalkraft und der anschließende Mischvorgang durch eine kreisende Hin- und Herbewegung des Behälters erfolgt. Die in einer solchen Vorrichtung zunächst auftretende, erhebliche Zentrifugalkraft kann dazu herangezogen werden, sowohl die Ventilanordnung zu öffnen als auch die erste Komponente restlos in die Mischkammer zu treiben. Ähnlich kann bei der für den anschließenden Mischvorgang zuständigen kreisförmigen Hin- und Herbewegung die auf das Mischgut jeweils einwirkende Beschleunigungskraft zum Schließen der Ventilanordnung ausgenutzt werden, so daß ein Entweichen von Mischgut oder Komponenten davon aus der Mischkammer verhindert wird. Die Schließ- und Dichtungsfunktion der Ventileinrichtung wird dabei durch deren eigene Vorspannung unterstützt.

Die Ausgestaltung nach Anspruch 2 ergibt eine besonders sichere gegenseitige Abdichtung der beiden Kammern im anfänglichen Lagerzustand ergibt.

Eine weitere Maßnahme zur sicheren Abdichtung der ersten Kammer im Lagerzustand, die insbesondere dann sinnvoll ist, wenn diese gemäß Anspruch 8 zur Aufnahme einer flüssigen Komponente dient, besteht in der nach Anspruch 3 vorgesehenen Folie. Dabei ergibt sich eine besonders einfache Handhabung zur Freigabe der Verbindung zwischen den beiden Kammern in der Weiterbildung der Erfindung nach Anspruch 4.

Die Maßnahme des Anspruchs 5 ist insofern von Vorteil, als sie dazu dient, die die erste Kammer abdichtende Folie im Durchstechungsbereich festzuklemmen, wodurch die Gefahr vermieden wird, daß Teile der Folie in das Gemisch gelangen.

Die Gestaltung nach Anspruch 6 dient dazu, die durchstochene Folie so zu verformen, daß die Flüssigkeit bei Auftreten der erwähnten Kraftwirkung mit Sicherheit dem durchstochenen Bereich zugeführt wird.

Die Maßnahmen des Anspruchs 7 sind vom Standpunkt der einfachen Ausbringung des fertigen Gemisches unmittelbar aus der Mischkammer von Vorteil.

Ein Ausführungsbeispiel der Erfindung wird nachstehend anhand der Zeichnungen näher erläutert. Darin zeigt
Figur 1 einen Längsschnitt durch einen Zweikammerbehälter in einer ersten Stellung, die dem Lagerzustand entspricht,
Figur 2 bis 5 jeweils der Figur 1 ähnliche Längsschnitte durch den Behälter in einer zweiten, aktivierten Stellung, wobei Figur 2 den Ausgangs-Ruhezustand, Figur 3 den Zustand unter Einwirkung einer Zentrifugalkraft zum Zusammenführen der beiden Komponenten, Figur 4 den Zustand während einer Mischphase und Figur 5 den Zustand während einer Kompressionsphase veranschaulicht, und
Figur 6 eine schematische Darstellung eines Zentrifugier- und Mischgerätes, wie es sich insbesondere zur Ausführung der verschiedenen Arbeitsphasen gemäß Figur 3 bis 5 eignet.

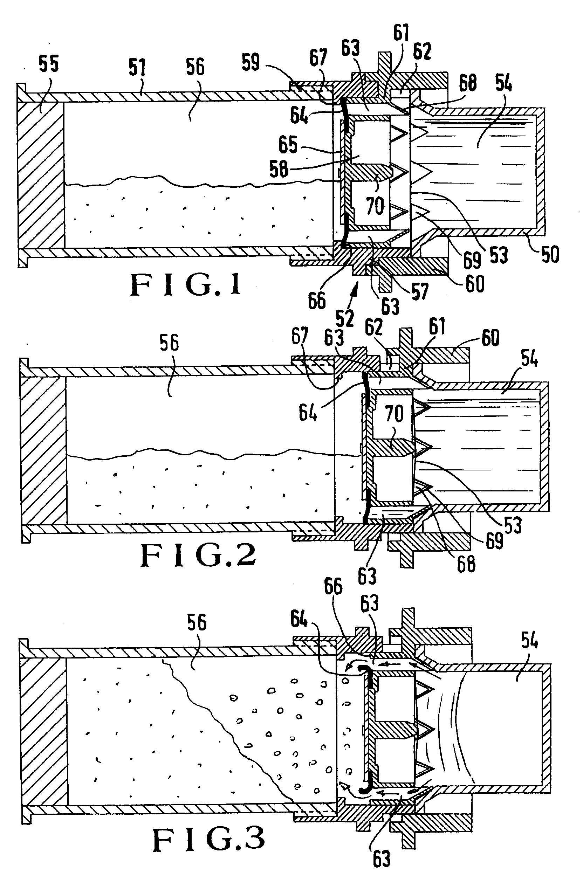

Der in Figur 1 gezeigte Behälter besteht im wesentlichen aus zwei zylindrischen Behälterteilen 50, 51 und einer dazwischen befindlichen Trenneinrichtung 52. Der Behälterteil 50 ist becherförmig gestaltet, an seiner der Trenneinrichtung 52 zugewandten Stirnseite durch eine Folie 53 verschlossen und bildet eine erste Kammer 54 zur Aufnahme einer beispielsweise flüssigen Komponente. Der Behälterteil 51 ist rohrförmig gestaltet, an seiner der Trenneinrichtung 52 zugewandten Stirnseite offen sowie an seiner entgegengesetzten Stirnseite durch einen verschiebbaren Kolben 55 verschlossen und bildet eine zweite Kammer 56 zur Aufnahme einer beispielsweise pulverförmigen Komponente. Die zweite Kammer 56 bildet zugleich die Mischkammer des Behälters.

Bei den genannten Komponenten kann es sich insbesondere um die Bestandteile eines Knochenzementes handeln, für dessen Herstellung der hier beschriebene Behälter besonders geeignet ist.

Die Trenneinrichtung 52 umfaßt eine zylindrische Zwischenhülse 57 und ein gegenüber dieser in Axialrichtung des Behälters verschiebbares Aktivierungsteil 58. Die Hülse 57 ist an ihren beiden Stirnseiten mit den Behälterteilen 50 und 51 verbunden, wobei die Verbindung mit dem Behälterteil 51 lösbar ist und beispielsweise ein Gewinde 59 umfaßt.

Um das Aktivierungsteil 58 relativ zu der Hülse 57 und damit zu dem Behälterteil 50 verschieben zu können, ist ein äußerer Ring 60 vorgesehen, der über mehrere am Umfang verteilte Stege 61 mit dem Aktivierungsteil verbunden ist; die Stege 61 durchsetzen in der Hülse 57 vorgesehene Schlitze 62.

Das im wesentlichen becherförmige Aktivierungsteil 58 weist in seinem Randbereich mehrere über den Umfang gleichmäßig verteilte, in Axialrichtung verlaufende Durchführungen 63 auf. An dem der Mischkammer 56 zugewandten Ende sind die Durchführungen 63 durch eine Dichtscheibe 64 verschlossen, die mittels einer Halteplatte 65 zentrisch an dem Aktivierungsteil 58 eingespannt ist. In der in Figur 1 gezeigten Ruhestellung ist der äußere Rand der Dichtscheibe 64 zwischen einem vom Außenrand des Aktivierungsteils 58 gebildeten Dichtungssitz 66 und einem inneren Ringflansch 67 der Hülse 57 in einer die Durchführungen 63 versperrenden Stellung verriegelt.

An der dem Behälterteil 50 zugewandten Seite endet jede Durchführung 63 an der Innenseite eines Dorns 68, der etwa die Form eines halben Hohlkegels hat. In der Schnittdarstellung der Figur 1 sind fünf Dorne 68 zu sehen, von denen die beiden äußeren geschnitten sind. Insgesamt sind acht Dorne 68 entsprechend acht Durchführungen 63 über den Umfang des Aktivierungsteils 58 verteilt vorgesehen.

In der in Figur 1 gezeigten Ruhestellung befinden sich die Spitzen der Dorne 68 in Abstand von der Folie 53. Wird nun das Aktivierungsteil 58 durch Erfassen des äußeren Rings 60 - sei es von Hand oder mit Hilfe einer geeigneten Vorrichtung - in die in Figur 2 gezeigte Aktivierungsstellung verschoben, so durchstoßen die Dorne 68 die Folie 53, wobei die halbkegeligen Außenflächen der Dorne 68 in komplementär gestaltete, halbkegelförmige Ausnehmungen 69 in der Innenfläche des Behälterteils 50 eingreifen. Die Folie 53 wird dabei in den Durchstoßungsbereichen zwischen den Außenflächen der Dorne 68 und den Ausnehmungen 69 eingeklemmt und festgehalten, wodurch die in der Folie erzeugten Löcher offengehalten und außerdem vermieden wird, daß Teile der Folie frei werden und in das Gemisch gelangen.

In dieser Stellung ist die Dichtung 64, 66 noch geschlossen, so daß die Komponenten noch immer voneinander getrennt sind. Der so erreichte Zwischenzustand kann für die praktische Handhabung wichtig sein, wenn zwischen dem beschriebenen Aktivierungsschritt und dem Mischvorgang ein längerer Zeitraum liegt, in dem die Komponenten noch nicht miteinander reagieren dürfen. Der Mischvorgang, der einschließlich der anfänglichen Überführung der flüssigen Komponente in die Mischkammer 56 und einschließlich einer abschließenden Verdichtung zum Entlüften der fertig gemischten Substanz in dem weiter unten beschriebenen Gerät ausgeführt wird, kann daher erst unmittelbar vor dem Einsatz der Substanz eingeleitet werden, während alle vorbereitenden Arbeiten auch länger vorher erledigt werden können.

Wie ferner in Figur 2 gezeigt, drückt in der aktivierten Stellung ein an dem Aktivierungsteil 58 zentrisch vorgesehener Stößel 30 gegen den mittleren Bereich der Folie 53 und lenkt sie in Richtung der Kammer 54 aus. Die Folie 53 wird dadurch in eine insgesamt flach kegelförmige Form gezwungen, die dazu beiträgt, daß bei Auftreten einer die Flüssigkeit in die Mischkammer 56 überführenden Kraft diese Flüssigkeit den Durchführungen 63 zugeleitet wird und sich keine mittlere Vertiefung bilden kann, in der sich ein Flüssigkeitsrest sammeln würde.

In der in Figur 2 gezeigten Aktivierungsstellung ist die Dichtscheibe 64 aufgrund ihrer Einspannung und Elastizität noch gegen den Dichtungssitz 66 vorgespannt und trennt die beiden Kammern 54 und 56 voneinander, obwohl die Verriegelung durch den Ringflansch 67 aufgehoben ist. Wirkt nun auf den Behälter eine gemäß der Darstellung von rechts nach links gerichtete Beschleunigung, so löst sich die Dichtscheibe 64 von dem Dichtungssitz 66 aufgrund der von der Flüssigkeit auf sie ausgeübten Kraft, die von der eigenen Massenträgheit der Dichtscheibe 64 unterstützt wird. In dieser Stellung wird die Flüssigkeit aus der Kammer 54 in die Mischkammer 56 überführt, wie dies in Figur 3 angedeutet ist.

Anschließend wird der Behälter in eine kreisende Hin- und Herbewegung versetzt, wobei die beiden Komponenten eine Wälzbewegung längs der Innenwand der Mischkammer 56 ausführen und innig miteinander vermischt werden. Während dieser kreisenden Hin- und Herbewegung wird die Dichtscheibe 64 aufgrund ihrer Vorspannung gegen den Dichtungssitz 66 gepreßt, wobei die Dichtkraft durch die auftretenden Beschleunigungskräfte immer dann unterstützt wird, wenn sich auch das Gemisch in Richtung der Dichtung bewegt. Dadurch wird ein Rückfließen von Teilen des Gemisches oder seiner Komponenten aus der Mischkammer 56 in die Kammer 54 verhindert. Die in der Wand der Mischkammer 56 angeordnete Dichtung 64, 66 arbeitet somit als Ventileinrichtung.

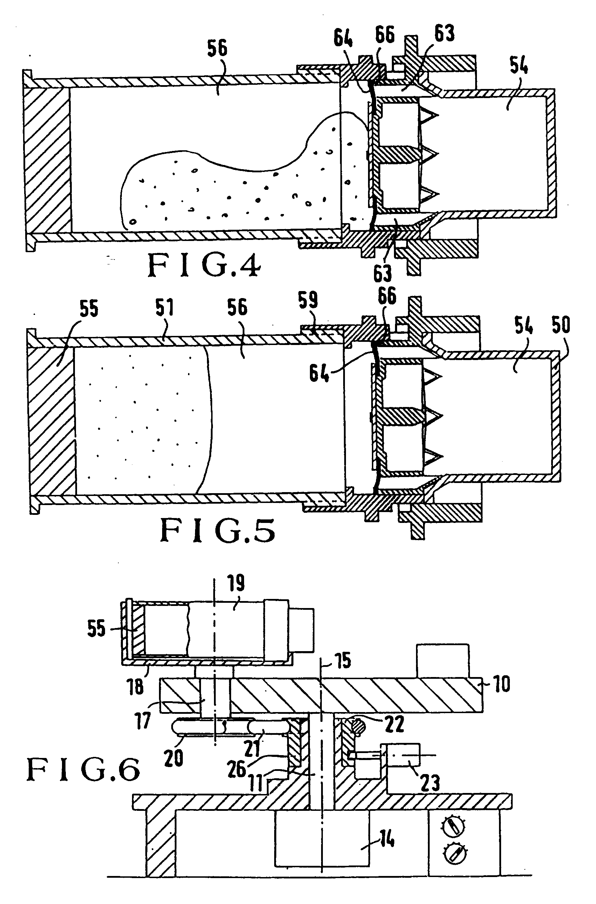

In Figur 5 ist schließlich eine an die Mischphase anschließende Kompressionsphase veranschaulicht, bei der der Behälter erneut einer von rechts nach links gerichteten Beschleunigung ausgesetzt wird, um die fertig gemischte Substanz zu verdichten und beim Mischen eingebrachte Luft herauszupressen.

Das in Figur 6 gezeigte Zentrifugier- und Mischgerät weist einen von einem Motor 14 über eine Welle 11 angetriebenen Rotor 10 auf, der einen auf einer Exzenterwelle 17 drehbaren Halter 18 zur Aufnahme eines Behälters gemäß Figur 1 bis 5 trägt. Der Behälter ist in Figur 6 mit 19 bezeichnet Die Exzenterwelle 17 trägt eine Riemenscheibe 20, die über einen Riemen 21 mit einer um die Achse 15 der Rotorwelle 11 drehbar gelagerten Riemenscheibe 22 gekoppelt ist. An einer unteren Verlängerungshülse 26 der Riemenscheibe 22 greift eine Kupplung 23 an.

Ist die Kupplung 23 ausgerückt, so kann sich der Behälter 19 auf eine relativ zu dem Rotor 10 feste Orientierung einstellen, in der der Kolben 55 radial nach außen weist. In dieser Orientierung wird der Behälter 19 einer reinen Zentrifugalkraft ausgesetzt, die die oben erwähnte von rechts nach links gerichtete Beschleunigungskraft darstellt und zur Überführung der flüssigen Komponente aus der Kammer 54 in die Mischkammer 56 gemäß Figur 3 sowie anschließend an die Mischphase zur Durchführung des Kompressionsvorgangs gemäß Figur 5 dient.

Befindet sich die Kupplung 23 dagegen in der in Figur 6 gezeigten eingerückten Stellung, so werden die Hülse 26 und damit die Riemenscheibe 22 stationär gehalten, wodurch nun bei fortgesetzter Drehung des Rotors 10 der Behälter 19 in Drehung um die Achse der Welle 17 versetzt wird. Dadurch ergibt sich die für den Mischvorgang nach Figur 4 benötigte kreisende Hin- und Herbewegung. Die Kupplung 23 kann bei fortgesetzter Rotordrehung ein- und ausgerückt werden, so daß die anhand von Figur 3 bis 5 geschilderten Arbeitsphasen unmittelbar aufeinander folgend durchgeführt werden können.

Wie aus der obigen Erläuterung ersichtlich, erfolgt nach der Aktivierung des Behälters dessen weitere Handhabung bis zur Fertigstellung der Substanz vollautomatisch mit Hilfe des Zentrifugier- und Mischgeräts nach Figur 6, wobei Fehlbedienungen ausgeschlossen sind.

## Patentansprüche

1. Behälter für durch Mischen von Komponenten herzustellende Substanzen mit
einer ersten Kammer (54) zur Aufnahme einer ersten Komponente,
einer eine Mischkammer bildenden zweiten Kammer (56) zur Aufnahme einer zweiten Komponente und
einer zwischen den Kammern angeordneten Trenneinrichtung (52), die die Kammern (54, 56) im Lagerzustand gegeneinander verschließt und zur Einleitung einer Mischphase so betätigbar ist, daß sie die Überführung der ersten Komponente in die Mischkammer (56) gestattet, und die eine in der Wand der Mischkammer (56) angeordnete Ventileinrichtung (64, 66) umfaßt,
dadurch gekennzeichnet, daß die Ventileinrichtung (64, 66) in ihrer zur Richtung der Überführung der ersten Komponente von der ersten zur zweiten Kammer (54, 56) entgegengesetzten Schließrichtung federnd vorgespannt ist und eine elastische Dichtscheibe (64) aufweist, die in der Trenneinrichtung (52) eingespannt ist und mit ihrem freien Rand an einem Dichtungssitz (66) anliegt.

2. Behälter nach Anspruch 1, gekennzeichnet durch ein Sperrelement (67), das in einer im Lagerzustand eingenommenen Verriegelungsstellung den Rand der Dichtscheibe (64) gegen den Dichtungssitz (66) drückt, wobei die Verriegelungstellung durch eine Relativverschiebung zwischen Sperrelement (67) und Dichtscheibe (64) in eine Freigabestellung überführbar ist.

3. Behälter nach Anspruch 1 oder 2, dadurch gekennzeichnet, daß die erste Kammer (54) im Lagerzustand durch eine zur Einleitung der Mischphase durchstechbare Folie (53) verschlossen ist.

4. Behälter nach Anspruch 3, soweit dieser von Anspruch 2 abhängt, dadurch gekennzeichnet, daß die Trenneinrichtung (52) einen Dorn (68) zum Durchstechen der Folie (53) aufweist, der an einem die Dichtscheibe (64) und den Dichtungssitz (66) tragenden Bauteil (58) angeordnet und mit diesem relativ zu dem Sperrelement (67) und der Folie (53) verschiebbar ist.

5. Behälter nach Anspruch 4, dadurch gekennzeichnet, daß der Dorn (68) ein eine Durchführung (63) in dem Bauteil (58) teilweise umgebendes Wandelement aufweist, dessen Außenfläche in der Freigabestellung in einen in der ersten Kammer (54) vorgesehenen, komplementär geformten Flächenteil (69) eingreift.

6. Behälter nach Anspruch 5, dadurch gekennzeichnet, daß das Bauteil (58) einen Stößel (70) trägt, der in der Freigabestellung die Folie (53) an einer dem Durchstechungsbereich gegenüberliegenden Stelle in Richtung der ersten Kammer (54) auslenkt.

7. Behälter nach einem der Ansprüche 1 bis 6, dadurch gekennzeichnet, daß die Mischkammer (56) zum Ausbringen der fertig gemischten Substanz von der Trenneinrichtung (52) lösbar und an ihrem der Trenneinrichtung (52) gegenüberliegenden Ende durch einen Ausbringkolben (55) verschlossen ist.

8. Behälter nach einem der Ansprüche 1 bis 7, dadurch gekennzeichnet, daß die erste Kammer (54) zur Aufnahme einer flüssigen und die zweite Kammer (56) zur Aufnahme einer pulverförmigen Komponente, insbesondere eines Knochenzements, dient.

## Claims

1. A container for substances to be prepared by mixing components, comprising
a first chamber (54) for holding a first component,
a second chamber (56) for holding a second component and forming a mixing chamber, and
partitioning means (52) disposed between the chambers for sealing the chambers (54, 56) against each other in a storage condition, the partitioning means being operable to permit the first component to be transferred to the mixing chamber (56) for initiating a mixing phase, and including valve means (64, 66) disposed in the wall of the mixing chamber (56),
characterised in that the valve means (64, 66) is resiliently biased in its closing direction opposite to the direction of transfer of the first component from the first to the second chamber (54, 56), and includes a resilient gasket (64) which is clamped in the partitioning means (52) with its free peripheral portion engaging a sealing seat (66).

2. The container of claim 1, characterised by a locking member (67) which, in a locking position taken in the storage condition, presses the peripheral portion of the gasket (64) against the sealing seat (66), the locking position being capable of being changed into a release position by a relative movement between the locking member (67) and the gasket (64).

3. The container of claim 1 or 2, characterised in that the first chamber (54) in the storage condition, is closed by a sheet (53) which is arranged to be perforated for initiating the mixing phase.

4. The container of claim 3 as dependent on claim 2, characterised in that the partitioning means (52) includes a spike (68) for perforating the sheet (53), the spike being disposed on a structural part (58) which carries the gasket (64) and the sealing seat (66) and is movable with the structural part relative to the locking member (67) and the sheet (53).

5. The container of claim 4, characterised in that the spike (68) includes a wall member partly surrounding a passage (53) in the structural part (58), the outer surface of the wall member engaging, in the release position, a complementarily shaped surface portion (69) provided in the first chamber (54).

6. The container of claim 5, characterised in that the structural part (58) carries a plunger (70) which, in the release position, deflects the sheet (53) at a position opposite the area of perforation toward the first chamber (54).

7. The container of any of claims 1 to 6, characterised in that the mixing chamber (56) is detachable from the partitioning means (52) and has its end opposite from the partitioning means (52) closed by a dispensing piston (55). for dispensing the finished mixed substance.

8. The container of any of claims 1 to 7, characterised in that the first chamber (54) serves to hold a liquid component and the second chamber (56) serves to hold a powdery component, specifically of a bone cement.

## Revendications

1. Récipient pour substances préparées par mélange de composants, comprenant une première chambre (54) pour la réception d'un premier composant, une seconde chambre (56) formant chambre de mélange et destinée à recevoir le second composant, et un dispositif de séparation (52) qui se situe entre les deux chambres et ferme les chambres (54, 56) l'une par rapport à l'autre à l'état de stockage, qui peut être actionné pour le déclenchement d'une phase de mélange de façon à permettre l'amenée du premier composant dans la chambre de mélange (56) et qui comprend un système de soupape (64, 66) disposé dans la paroi de la chambre de mélange (56), caractérisé en ce que le système de soupape (64, 66) est mis en précontrainte élastique dans sa direction de fermeture opposée à la direction de transport de la première vers la seconde chambre (54, 56) et comporte une rondelle d'étanchéité (64) enserrée dans le dispositif de sépa-ration (52) et appliquée avec son bord libre contre un siège d'étanchéité (66).

2. Récipient selon la revendication 1, caractérisé en ce qu'il comprend un élément d'arrêt (67) qui, dans une position de verrouillage occupée à l'état de stockage, applique le bord de la rondelle d'étanchéité (64) contre le siège d'étanchéité (66), le passage de la position de verrouillage à une position de libération étant obtenu par un déplacement relatif entre l'élément d'arrêt (67) et la rondelle d'étanchéité (64).

3. Récipient selon l'une des revendications 1 ou 2, caractérisé en ce que, à l'état de stockage, la première chambre (54) est fermée par une feuille (53) qui peut être percée pour déclencher la phase de mélange.

4. Récipient selon la revendication 3, dans la mesure où celle-ci dépend de la revendication 2, caractérisé en ce que le dispositif de séparation (52) comprend une pointe (68) pour le percement de la feuille (53), qui est montée sur un élément (58) portant la rondelle d'étanchéité (64) et le siège d'étanchéité (66) et qui peut être déplacée avec ledit élément par rapport à l'élément d'arrêt (67) et à la feuille (53).

5. Récipient selon la revendication 4, caractérisé en ce que la pointe (68) comporte un élément de paroi qui entoure un passage (63) dans l'élément (58) et dont la surface extérieure s'engage dans la position de libération dans un élément de surface (69) de forme complémentaire prévu dans la première chambre (54).

6. Récipient selon la revendication 5, caractérisé en ce que l'élément (58) porte un poinçon (70) qui, dans la position de libération, fait dévier la feuille (53) en un point en face de la zone de percement, en direction de la première chambre (54).

7. Récipient selon l'une quelconque des revendications 1 à 6, caractérisé en ce que, pour la distribution de la substance entièrement mélangée, la chambre de mélange (56) peut être retirée du dispositif de séparation (52) et que son extrémité opposée audit dispositif de séparation (52) est fermée par un piston de distribution (55).

8. Récipient selon l'une quelconque des revendications 1 à 7, caractérisé en ce que la première chambre (54) sert à recevoir un composant liquide et que la seconde chambre (56) est prévue pour recevoir un composant pulvérulent, en particulier un ciment d'os.
